# EUROPEAN PATENT SPECIFICATION

(11) **EP 1 926 738 B1**
(45) Date of publication and mention of the grant of the patent: **09.12.2009**
(21) Application number: 06793551.0
(22) Date of filing: 15.09.2006
(51) Int. Cl.: C07D 495/04

(54) **SYNTHESIS OF CYCLOPENTADIENEDITHIOPHENE DERIVATIVES**
SYNTHESE VON CYCLOPENTADIENDITHIOPHEN-DERIVATEN
SYNTHÈSE DE DÉRIVÉS DE CYCLOPENTADIÈNEDITHIOPHÈNE

(30) Priority: 19.09.2005 EP 05108625; 23.09.2005 US 720024 P
(43) Date of publication of application: 04.06.2008
(73) Proprietor: Basell Polyolefine GmbH, 50389 Wesseling (DE)
(72) Inventor: GUIDOTTI, Simona, I-40051 Bologna (IT)
(74) Representative: Sacco, Marco
(86) International application number: PCT/EP2006/066400
(87) International publication number: WO 2007/033937

(56) References cited:
- WO-A2-02/092564
- GOTTELAND J-P ET AL: "Sulfonamide Derivatives of Benzylamine Block Cholesterol Biosynthesis in HepG2 Cells : A New Type of Potent Squalene Epoxidase Inhibitors" BIOORGANIC & MEDICINAL CHEMISTRY LETTERS, OXFORD, GB, vol. 8, no. 11, 2 June 1998 (1998-06-02), pages 1337-1342, XP004137200 ISSN: 0960-894X

## Description

The present invention relates to a process for the preparation of cyclopentadiene derivatives of formula (I) 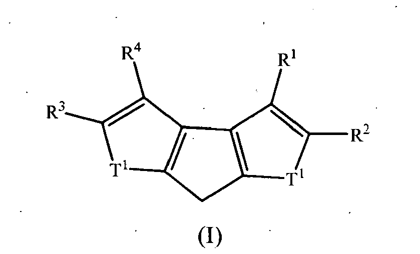 wherein T¹ is a sulfur or an oxygen atom, and R¹, R², R³ and R⁴ are hydrogen atoms or C¹-C⁴⁰ hydrocarbon radicals, or R¹ and R² and/or R³ and R⁴ can join together to form a condensed ring.

Cyclopentadiene derivatives containing heterocyclic fused ring are used as starting point for the synthesis of metallocene compounds, which are well known in the art as catalyst components for the polymerization of olefins. This class of metallocene compounds is disclosed for example in WO 98/22486.

WO 02/092564 relates to a three step process for the preparation of cyclopentadiene derivative of formula

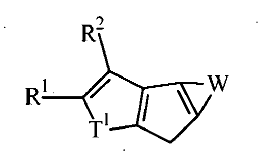

In step a) of this process a compound of formula (II) 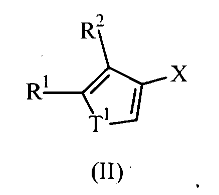 is reacted with a compound of formula (III) 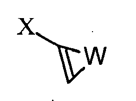 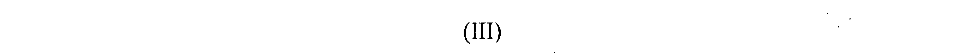 wherein in particular X is selected from the group consisting of chlorine, iodine, bromine. Therefore this document relates to a process in which the coupling step is carried out starting from two halogen substituted compounds.
Even if this process is claimed to be very efficient, the applicant unexpected discovered that there is the possibility to improve the yield of the over process by using in step a) different starting substances in order to achieve the compound of formula (IV) described in WO 02/092564.

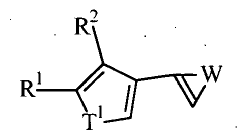

From another point of view the coupling of two substituted thiofene rings, wherein the first thiofene ring is substituted in position 3 by an halogen atom and the second thiofene ring is substituted in position 3 by a boron derivative is known in Israel Journal of Chemistry vol. 27 1986 pp 25-28. However the yields for this reaction reported in this document are quite low, they range from 65% to 36%. All the thiofene derivatives used are halogen substituted. Another Suzuki coupling using 3-thiophene boronic acid is yields between 50 and 80% is known from Bioorganic and Medicinal Chemistry Letters 8, 1998, 1337-1342. This means that in view of this document this reaction is clearly considered not useful in order to improve the yield of the process described in WO 02/092564.

The applicant unexpected found that when the thiofene rings are substituted by hydrocarbon groups or they are unsubstituted the yield of the latter reaction is considerably improved, so that it can be used in order to improved the overall yield of the process described in WO 02/092564.

The first object of the present invention is a process for preparing cyclopentadiene derivatives having formula (I) 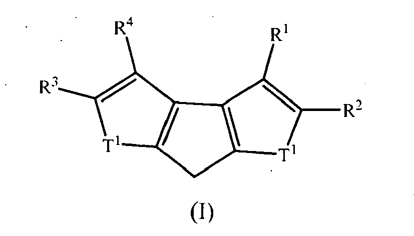 wherein
T¹ is selected from the group consisting of oxygen (O), or sulphur (S);
R¹, R², R³ and R⁴, equal to or different from each other, are hydrogen atoms, C₁-C₄₀ hydrocarbon radicals or R¹ and R² and/or R³ and R⁴ can join to form one or more C₃-C₆ aromatic or aliphatic fused rings; preferably R¹, R², R³ and R⁴, equal to or different from each other, are hydrogen atoms or linear or branched, cyclic or acyclic, C₁-C₂₀-alkyl, C₂-C₂₀ alkenyl, C₂-C₂₀ alkynyl, C₆-C₂₀-aryl, C₇-C₂₀-alkylaryl or C₇-C₂₀-arylalkyl radicals.

More preferably R⁴ and R¹ are hydrogen atoms and R³ and R² are linear or branched, cyclic or acyclic, C₁-C₂₀-alkyl radicals; even more preferably linear or branched C₁-C₁₀ alkyl radicals such as methyl or ethyl radicals;
said process comprises the following steps:
a) reacting a compound of formula (II) 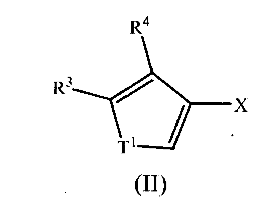 with a compound of formula (III) 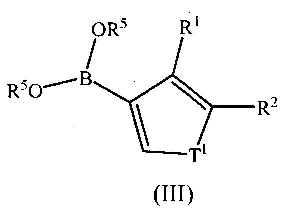 in the presence of a palladium or nickel based catalyst and a base;
   wherein T¹, R¹, R², R³ and R⁴ have the above indicated meaning, X is selected from the group consisting of chlorine, iodine, bromine; and R⁵, equal to or different from each other, is a hydrogen atoms, linear or branched, cyclic or acyclic, C₁-C₂₀-alkyl, C₂-C₂₀ alkenyl, C₂-C₂₀ alkynyl, C₆-C₂₀-aryl, C₇-C₂₀-alkylaryl or C₇-C₂₀-arylalkyl radicals; preferably R⁵ is an hydrogen atom or a linear or branched, cyclic or acyclic, C₁-C₂₀-alkyl radical;
b) contacting the compound of formula (IV) 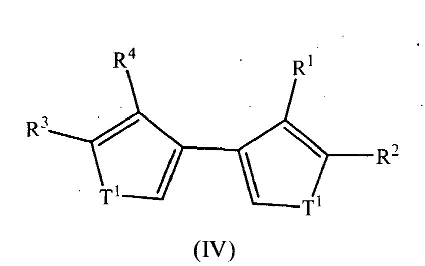 obtained from step a) with a carbonylating system; in order to obtain a compound of formula (IVa) 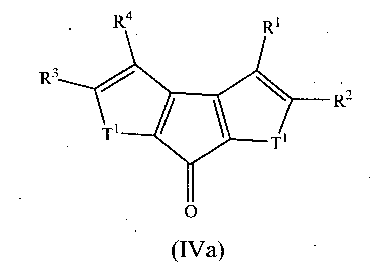 and
c) treating the product obtained in step b) with a reducing agent.

In step a) the palladium or nickel based catalyst are palladium metal, palladium compounds and/or nickel compounds. The catalysts can also have been applied to a solid support such as activated carbon or aluminum oxide. Preferably compound in which palladium is present in the oxidation state (0) are used. Examples of these compounds are palladium ketonates, palladium acetylacetonates, nitrilepalladium halides, palladium halides, allylpalladium halides and/or palladium biscarboxylates. Particularly preferred catalysts are palladium bisacetylacetonate, bis(benzonitrile)palladium dichloride, PdCl₂, Na₂PdCl₄, Na₂PdCl₆, bis(acetonitrile)palladium dichloride, palladium-II-acetate, bis(triphenylphosphine-)palladium dichloride, tetrakis(triphenylphosphine-)palladium, bis(diphenylphosphino)ferrocene-palladiumdichloride and/or tctrachloropalladic acid. The palladium compound can also be generated in situ, for example palladium(II) acetate from palladium(II) chloride and NaOAc.

The amount of catalyst preferably used is from 0.001 to 0.5 mol % and particularly preferably from 0.01 to 0.2 mol% with respect to the compound (II).

The catalyst can contain phosphorus-containing ligands or phosphoms-containing ligands can be added separately to the reaction mixture.

Preferred phosphorus-containing ligands are tri-n-alkylphosphines, triarylphosphines, dialkylarylphosphines, alkyldiarylphosphines and/or heteroarylphosphines such as tripyridylphosphine and trifurylphosphine, where the three substituents on the phosphorus can be identical or different and one or more substituents can link the phosphorus groups of two or more phosphines, with part of this linkage also being able to be a metal atom. Particular preference is given to phosphines such as triphenylphosphine, tri-tert-butylphosphine, tricyclohexylphosphine, bis(diphenylphosphino)ferrocene and/or tris-(3-sulfophenyl)phosphine trisodium salt (TPPTS).

The total concentration of phosphorus-containing ligands is, based on the compound of formula (II), preferably up to 1 mol %, most preferably from 0.001 to 1 mol % and in particular from 0.01 to 0.5 mol%.

The bases usually used in process step a) are alkali metal hydroxides, akaline earth metal hydroxides, alkali metal carbonates, alkaline earth metal carbonates, alkali metal hydrogencarbonates, alkali metal alkoxides, alkaline earth metal alkoxides, alkali metal fluorides, primary amines, secondary amines or tertiary amines. Preference is given to bases such as sodium hydroxide, potassium hydroxide, lithium carbonate, sodium carbonate, potassium carbonate or potassium fluoride. Mixtures of bases can also be used. The amount of base used is preferably 1-10, particularly preferably 1-5 and in particular 1-2.5 mol-equivalents of base, based on the aromatic (II).

Solvents preferably used in step a) are alcohols, ethers, polyethylene glycols, sulfoxides, formamides, or mixtures thereof Preferred solvents are dimethyl sulfoxide (DMSO), dimethyl fonnamide (DMF), 1,2-dimethoxyethane (DME) and tethahydrofurane (THF) or mixtures thereof In all cases, water, 1,2-dimethoxyethane, tetrahydrofuran or lipophilic solvents such as toluene, xylene, chlorobenzene or dichloromethane can be added as cosolvent.

Step a) is preferably carried out at a temperature ranging from 10°C to 150°C; more preferably from 40°C to 100°C; even more preferably from 70°C to 90°C.

Examples of palladium or nickel based catalyst can be found on "Miyaura N.; Suzuki, A. Chem. Commun. 1979, 866"; "Miyaura N. et al. Tetrahedron Letters 1979, 3437"; " Miyaura, N.; Suzuki, A. Chem. Rev. 1995, 95, 2457" ; "Suzuki, A. Journal of Organometallic Chemistry 1999, 576, 147"; "Hassan, J.; Sévignon, M.; Gozzi, C.; Schulz, E.; Lemaire, M. Chem. Rev. 2002, 102, 1359".

For the purpose of the present invention the carbonylating system is defined as reagent or a series of reagents that in one or more steps can close the five membered ring in order to obtain compound of formula (IVa). Carbonylating step b) depends from the carbonyl system used. A preferred step b) comprises the following substeps:
i) contacting the compound of formula (IV) obtained from step a) with two equivalents of a base; and
ii) treating the dianionic compound obtained from step i) with a compound of formula (V) 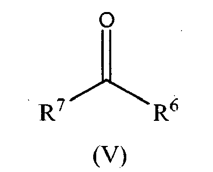 wherein R⁶ and R⁷ equal to or different from each other are selected from the group consisting of hydrogen, chlorine, bromine, iodine, OR and NR₂, wherein R is a C₁-C₂₀ hydrocarbon radical optionally containing heteroatoms belonging to groups 13-17 of the Periodic Table of the Elements; preferably R is a C₁-C₂₀-alkyl, C₃-C₂₀-cycloalkyl, C₆-C₂₀-aryl, C₇-C₂₀-alkylaryl, C₇-C₂₀-arylalkyl radical; preferably R⁷ is chlorine bromine, iodine CF₃, CCl₃ or OR, more preferably chlorine or OCH₂CH₃; preferably R⁶ is selected from the group consisting of CF₃, CCl₃, OR and NR₂, wherein R is described above, more preferably R⁶ is NR₂, even more preferably R⁶ is N(CH₃)₂;

An alternative embodiment for carrying out step b) comprises the following substeps:
i) contacting the compound of formula (IV) obtained from step a) with two equivalents of a base and subsequently with one equivalent of a compound selected from chlorine, bromine or iodine in order to obtain an anionic monohalogenated derivative; and
ii) treating the an anionic monohalogenated compound obtained from step i) with a compound of formula (Va)

   [MₘLⱼ(CCO)ₙ]^{a} (Va)
wherein M is a transition metal of groups 4-11 of the periodic table; L is a ligand that coordinates the metal M that can be neutral or with a positive or negative charge; a ranges from -4 to +4, it represents the charge of the complex when a is 0 the complex is neutral; m ranges from 1 to 20; j ranges from 0 to 30; and n ranges from 1 to 50; preferably a ranges from -2 to +2; m ranges from 1 to 10; j ranges from 0 to 5 and n ranges from 1 to 20.

A further alternative embodiment for carrying out step b) comprises the following substeps:
i) contacting the compound of formula (IV) obtained from step a) with a halogenating compound and subsequently with one equivalent of a base in order to obtain an anionic monohalogenated derivative; and
ii) treating the an anionic monohalogenated compound obtained from step i) with a compound of formula (Va)

   [MₘLⱼ(CO)ₙ]^{a} (Va)
wherein M is a transition metal of groups 4-11 of the periodic table; L is a ligand that coordinates the metal M that can be neutral or with a positive or negative charge; a ranges from -4 to +4 and represents the charge of the complex (when a is 0 the complex is neutral); m ranges from 1 to 20; j ranges from 0 to 30; and n ranges from 1 to 50; preferably a ranges from -2 to +2; m ranges from 1 to 10; j ranges from 0 to 5 and n ranges from 1 to 20.

The base used in step b) is preferably selected from hydroxides and hydrides of alkali- and alkaline-earth metals, metallic sodium and potassium and organometallic lithium compounds. Most preferably, said bases are methyllithium, n-butyllithium, or tertbutyllithium optionally activated with tetramethylethylene diamine (TMEDA).

Examples of halogenating compounds are described in "Comprehensive Organic Transformations" ed. 1989 VCH Publishers pages 315-318, such as for example chlorine, bromine, iodine CuCl₂, CBr₄, N-bromo-succinimide, N-chloro-succinimide.

Non limitative examples of compounds of formula (V) are:
carbethoxyimidazole, triphosgene, ethyl *N*,*N*-dimethylcarbamate, chloride of *N*,*N-*dimethylcarbamic acid.

Non limitative examples of ligands L are; halogen, hydrogen, nitrogen, amines, phosphine, cyclopentadienyl derivatives, octadienes.

Non limitative examples of compound of formula (Va) are C(CO)₆, Cr(CO)₆, Cr(CO)₅H₂, Mn(CO)₅H, Mn(CO)₅I, Mn₂(CO)₁₀, Mn₂(CO)₈H₂, Fe(CO)₅, Fe(CO)₄H₂, Fe(CO)₂X₂, Fe(CO)₄X₂, Fe(CO)₅X₂, Fe₂(CO)₉, Co(CO)₄H, Co(CO)₂X₂, Co(CO)₄X₂, Co(CO)₅X₂, Co₂(CO)₆, Ni(CO)₄, Ni₂(CO)₆H₂, Fe₃(CO)₁₂, [Fe₃(CO)₁₁H]⁻, Os₃(CO)₁₂, [Re₃(CO)₁₂H₆]⁻, [Re₄(CO)₁₆]²⁻, Co₄(CO)₁₂, [Fe₄(CO)₁₃]²⁻, Os₅(CO)₁₆, [Os₅(CO)₁₅I]⁻, [Ni₅(CO)₁₂]²⁻, [Fe₅C(CO)₁₅]⁻, Rh₆(CO)₁₆, Rh₆(CO)₁₅I, [Co₆(CO)₁₄]⁴⁻, [Fe₆C(CO)₁₆]²⁻, [Co₆H(CO)₁₅]⁻, [Rh₆C(CO)₁₅]²⁻, [Co₆N(CO)₁₅]⁻, Os₆(CO)₁₈;
wherein X is chlorine, bromine, iodine.

Step b) is carried out to a temperature range of from -78 C to 100°C preferably from -20°C to 30°C. Usually aprotic solvents are used such as diethyl ether, hexane, toluene, tetrahydrofuran, dimethoxyethane and dioxane. The product obtained from step b) is purified by process known in the art such as filtration, recrystallization, chromatography, distillation; or alternatively is used as such.

In step c) various reducing agent known in the art can be used. Examples of suitable reducing agent used in step c) are described in "Comprehensive Organic transformations" ed. 1989 VCH Publishers pages 35-40. Preferred reducing agents are LiAlH₄/AlCl₃ and N₂H₄/base, such as NaOH and KOH.

The solvent for carrying out step c) depends upon the reducing agent used. For example when LiAlH₄/AlCl₃ is used the reaction is carried out in an aprotic solvent either polar or apolar such as tetrahydrofuran, dimethoxyethane, diethyl ether, toluene, pentane, hexane. When N₂H₄/base is used a protic solvent such as water or diethylene glycol can also be used, optionally in the presence of a phase transfer agent. The temperature depends from the reducing agent used, it generally ranges from -80°C to 300°C, preferably from 0°C to 150°C.

Preferably the step c) comprises the following substeps:
i) contacting the compound of formula (IVa) with N₂H₄;
ii) adding a solution of KOH in water; and
iii) filtering the solid and recovering the product.

Step i) is preferably carried out in water, toluene or diethylene glycol; step ii) is preferably carried out in the presence of a phase transfer agent, preferably diethylene glycol.

The product obtained from step c) is purified by process known in the art such as filtration, crystallization, column chromatography, preferably by filtration.

The following examples are given to illustrate the invention.

### EXAMPLES

### General procedures.

All operations were performed under nitrogen by using conventional Schlenk-line techniques.

Solvents were purified by degassing with N₂ and passing over activated (8 hours, N₂ purge, 300 °C) Al₂O₃, and stored under nitrogen. 3-Bromothiophene (Aldrich, 97%), 3-thiopheneboronic acid (Aldrich), PPh₃ (Aldrich, 99%), KOH (Aldrich, 85%), Pd(OAc)₂ (Aldrich, 99.9%), 2-methylthiophene (Aldrich, 98%), B(OMe)₃ (Aldrich, 99.5) *n*-BuLi (Aldrich), were used as received.

The proton and carbon spectra of the obtained compounds were obtained on a Bruker DPX 200 spectrometer operating in the Fourier transform mode at room temperature at 200.13 MHz and 50.32 MHz respectively. The samples were dissolved in CDCl₃ or CD₂Cl₂. CDCl₃ (Aldrich, 99.8 atom % D) and CD₂Cl₂ (Aldrich, 99.5 atom %) were stored over molecular sieves (4-5 Å).

Preparation of the samples was carried out under nitrogen using standard inert atmosphere techniques. The residual peak of CHCl₃ or CHDCl₂ in the ¹H spectra (7.25 ppm and 5.35 ppm, respectively) and the peak of the solvent in the ¹³C spectra (77.00 ppm for CDCl₃ and 53.80 ppm for CD₂Cl₂) were used as a reference.

Proton spectra were acquired with a 15° pulse and 2 seconds of delay between pulses; 32 transients were stored for each spectrum. The carbon spectra were acquired with a 45° pulse and 6 seconds of delay between pulses; about 512 transients were stored for each spectrum.

GC-MS analyses were carried out on a HP 5890 - serie 2 gas-chromatograph and a HP 5989B quadrupole mass spectrometer.

### Example 1

### Step a) Synthesis of 3,3'-dithiophene

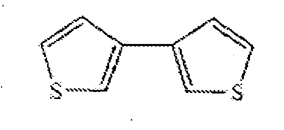

A 1000 mL three-necked round-bottom flask, equipped with a magnetic stirrer and a reflux condenser, was charged with 3-bromothiophene (0.092 mol, 8.6 mL, 1 eq.), 3-thiopheneboronic acid (0.110 mol, 14.08 g, 1.2 eq.), KOH (0.276 mol, 3 eq.), DME (d = 0.867, 3.07 mol, 319 mL) and H₂O (54 mL).

The reaction mixture was heated at 80°C. In a separate Schlenk flask, PPh₃ (7.36 mmol, 0.08 eq.) was dissolved in DME (59.8 mL) and then Pd(OAc)₂ (1.84 mmol, 0.02 eq.) was added. The catalytic solution was stirred for 15 min, to preactivate the catalyst, and then was added to the reaction mixture at 80°C. The reaction mixture turned immediately brown. The reaction took 9 h to completion. Reaction mixture was diluted with water, the two phases were separated and the aqueous phase was washed with toluene. The combined organic phases were washed with brine, with water and then dried on Na₂SO4 and MgSO₄. The solvents were eliminated under vacuum to give 13.88 g of a grey powder which was analyzed by GC-MS analysis and ¹H NMR spectroscopy. The GC analysis showed the presence of the desired product 3,3'-dithiophene.
Yield of pure product 76.9% ( 0.071 mol).
¹H NMR (δ, ppm, CD₂Cl₂): 7.50-7.36 (2 m, 6H).
¹³C NMR (δ, ppm, CD₂Cl₂): 120.19(*C*Hα); 126.56 (*C*Hβ); 126.71 (*C*Hδ); 137.61 (*Cq*).
m/z (%): 166 [M^{·+}], 134 (32), 121 (45).

### Example 2

### Step a) Synthesis of 2-methyl-4,3'-dithiophene

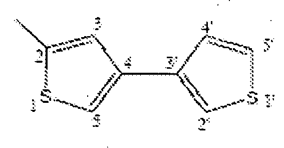

A 500 mL two-necked round-bottom flask, equipped with a magnetic stirrer and a reflux condenser, was charged with 2-methyl-4-bromothiophene (11.52 g, 65.06 mmol, 1 eq.), 3-thiopheneboronic acid (10.0 g, 8.15 mmol, 1.2 eq.), KOH (0.195 mol, 3 eq.), DME (d = 0.867, 1.799 mol, 187 mL), and H₂O (37.4 mL). The reaction mixture was heated at 80°C. In a separate Schlenk tube, PPh₃ (5.3 mmol, 0.08 eq.) was dissolved in DME (37.4 mL) and then Pd(OAc)₂ (1.3 mmol, 0.02 eq.) was added. The catalytic solution was stirred for 15 minutes, to preactivate the catalyst, and then was added to the reaction mixture at 80°C. The reaction mixture turned immediately brown. The reaction took 6 h to completion. Reaction mixture was diluted with water, the two phases were separated and the aqueous phase was washed with toluene. The combined organic phases were washed with brine, with water and then dried on Na₂SO₄ and MgSO₄.The solvents were eliminated under vacuum to give 10.82 g of an orange powder: the latter was extracted with *n*-pentane and dried in vacuum to give 9.26 g of a yellow powder which was analyzed by GC-MS analysis and ¹H NMR spectroscopy. Yield of pure product 75.7% (49.26 mmol).
¹H NMR (δ, ppm, CDCl₃): 7.29 (m, 3H); 7.12 (d, 1H); 7.00 (d, 1H); 2.51 (d, 3H, CH₃).
¹³C NMR (δ, ppm, CDCl₃): 15.70 (*C*H3); 117.90 (*C*H in 3); 119.77 (*C*H in 5'); 124.98 (*C*H in 5); 126.41 (*C*H in 2' and CH in 4'); 137.29 (*C* in 4); 137.90 (*C* in 3'); 140.72 (*C* in 2).
m/z (%): 182 [M^{·+} + 2] (38), 181 [M^{·+} + 1] (66), 180 [M^{·+}] (100), 179 (100), 153 (35), 147 (100), 135 (48).

### Example 3

### Step a) Synthesis of 2,2'-dimethyl-4,4'-dithiophene

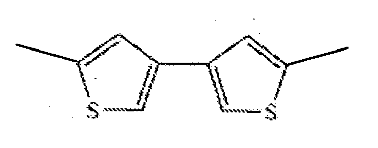

2-methyl-4-bromothiophene was prepared in two steps from 2-thiophenealdehyde following the procedure described in WO 02/092564. In the first step bromination of 2-thiophenealdehyde with Br₂/AlCl₃ gave 4-bromo-2-thiophenealdehyde, which was then reduced with NH₂NH₂/KOH/water to 2-methyl-4-bromothiophene: overall yield ca. 70% based on 2-thiophenealdehyde.

2-methyl-4-thiopheneboronic acid was prepared from 2-methyl-4-bromothiophene following the procedure reported in WO 95/02046 for the preparation of 3-thiopheneboronic acid.

### Synthesis of 2,2'-dimethyl-4,4'-dithiophene

A 250 mL three-necked round-bottom flask, equipped with a magnetic stirrer and a reflux condenser, was charged with 2-methyl-4-bromothiophene (3.54 g, 20.00 mmol, 1 eq.), 2-methyl-4-thiopheneboronic acid (3.33 g, 23.45 mmol, 1.2 eq.), KOH (59.00 mmol, 3 eq.), DME (d = 0.867, 0.554 mol, 57.6 mL), and H₂O (11.5 mL). The reaction mixture was heated at 80°C. In a separate Schlenk flask, PPh₃ (1.6 mmol, 0.08 eq.) was dissolved in DME (11.5 mL) and then Pd(OAc)₂ (0.39 mmol, 0.02 eq.) was added. The catalytic solution was stirred for 15 minutes, to preactivate the catalyst, and then was added to the reaction mixture at 80°C. The reaction mixture turned immediately brown. The reaction took 5 h to completion. Reaction mixture was diluted with water (200 mL), the two phases were separated and the aqueous phase was washed with toluene. The combined organic phases were washed with brine, with water and then dried on Na₂SO₄ and MgSO₄. The solvents were eliminated under vacuum at 40°C to give an orange powder: the latter was extracted with *n*-pentane and dried in vacuum to give 3.31 g of an orange powder which was analyzed by GC-MS analysis and ¹H NMR spectroscopy. Yield of pure product 85.2% (1.7.03 mmol).
¹H NMR (δ, ppm, CDCl₃): 7.04 (d, 2H); 6.94 (d, 2H); 2.48 (d, 6H, CH₃).
¹³C NMR (δ, ppm, CDCl₃): 15.67 (*C*H3); 117.53 (*C*H); 124.84 (*C*H); 137.60 (*Cq*); 140.50 (*Cq*). m/z (%): 194 [M^{·+}] (100), 193 [M^{·+} - 1] (32), 179 (6), 161 (18).

### Step b) Synthesis of 2,5-dimethyl-7H-cyclopenta[1,2-b:4,3-b']thiophen-7-one

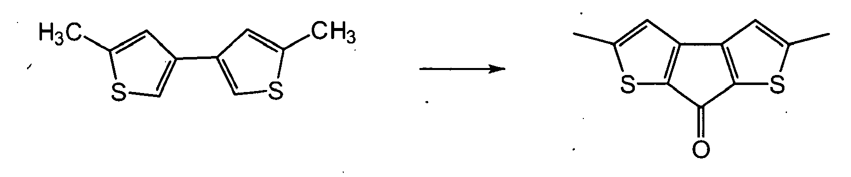

A mixture of 5.0 g (25.73 mmol) of 2,2'-dimethyl-4,4'-dithiophene, 50 mL of ether and 8.5 mL (56.61 mmol) of TMEDA was placed into the bulb, the resulting suspension was cooled to -20 °C and then treated with 25.75 mL (56.65 mmol) of 2.2 M BuLi in hexane. The mixture was allowed to warm up to r.t. and was stirred for 3 hours. The reaction mixture was treated with 3.0 g (25.73 mmol) of carbamate (EtO)C(O)NMe₂ in 70 mL of ether and then was stirred in 40 hours.

The resulting mixture was poured into 250 mL of saturated aqueous solution of NH₄Cl under shaking or stirring. Organic phase containing some of the precipitated product was isolated, washed with 125 mL of water, treated with 10 mL of hexane and then filtered. The precipitate was washed with water, twice with 10 mL of hexane and dried. Yield of first portion is 3.20 g. Filtrate was evaporated up to volume of 25 mL to give a suspension. This suspension was filtered, washed with hot hexane and dried. The yield of second portion is 1.45 g. Total yield 4.65 g (82%).
¹H NMR (CDCl₃): 6.58 (q, 1H); 2.50 (d, 6H).
¹³C NMR (CDCl₃): 178.8; 152.6; 152.2; 133.0; 118.6; 16.1.

### Step c) Reduction of 2,5-dimethyl-7H-cyclopenta[1,2-b:4,3-b']thiophen-7-one

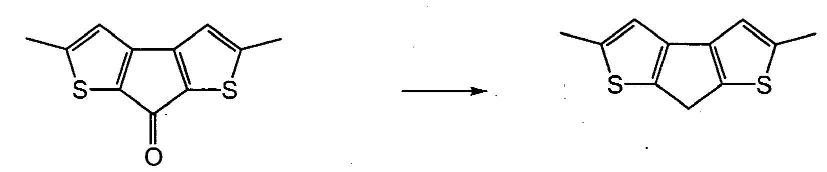

A mixture of 4.5 g (20.4 mmol) of 2,5-dimethyl-7*H*-cyclopenta[1,2-*b*:4,3-*b*']thiophen-7-one, 60 mL of diethylene glycol and 7 mL of hydrazine monohydrate was placed into the bulb. The mixture was wanned to 80°C and kept at this temperature in 1 h at stirring. Then the mixture was intensively refluxed in 1 h, cooled to r.t. and treated with a solution of 7 g KOH in 25 mL of water. The resulting mixture was carefully heated and the gas started to bubble. The mixture was refluxed for 2 hours, then it was cooled to 70÷80°C and poured into 300 mL water. The precipitate was decanted, filtered, washed 7 times with 20÷30 mL of water and dried. Yield 3.3 g (78%).
¹H NMR (CDCl₃): 6.81 (q, 2H); 3.72 (s, 2H); 2.58 (d, 6H).
¹³C NMR (CDCl₃): 143.6; 142.1; 140.1; 116.4; 33.1; 15.9.

## Claims

1. A process for preparing cyclopentadiene derivatives having formula (I) 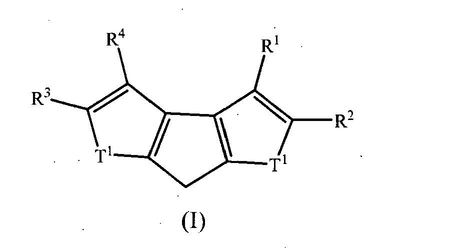 wherein
T¹ is selected from the group consisting of oxygen (O), or sulphur (S);
R¹, R², R³ and R⁴, equal to or different from each other, are hydrogen atoms, C₁-C₄₀ hydrocarbon radicals or R¹ and R² and/or R³ and R⁴ can join to form one or more C₃-C₆ aromatic or aliphatic fused rings;
comprising the following steps:
a) reacting a compound of formula (II) 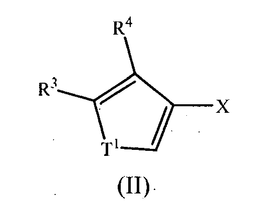 with a compound of formula (III) 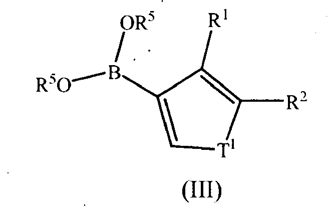 in the presence of a palladium or nickel based catalyst and a base;
wherein T¹, R¹, R², R³ and R⁴ have the above indicated meaning, X is selected from the group consisting of chlorine, iodine, bromine; and R⁵, equal to or different from each other, is a hydrogen atoms, linear or branched, cyclic or acyclic, C₁-C₁₀-alkyl, C₂-C₂₀ alkenyl, C₂-C₂₀ alkynyl, C₆-C₂₀-aryl, C₇-C₂₀-alkylaryl or C₇-C₂₀-arylalkyl radicals;
b) contacting the compound of formula (IV) 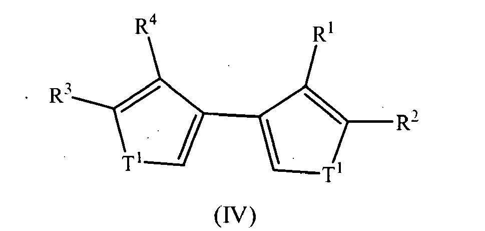 obtained from step a) with a carbonylating system; in order to obtain a compound of formula (IVa) 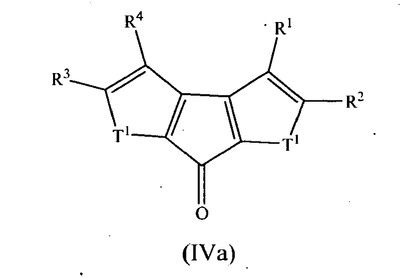 and
c) treating the product obtained in step b) with a reducing agent.

2. The process according to claim 1 wherein in step a) compound in which palladium is present in the oxidation state (0) are used as catalyst.

3. The process according to claim 2 wherein the palladium based catalyst is selected from palladium ketonates, palladium acetylacetonates, nitrilepalladium halides, palladium halides, allylpalladium halides and/or palladium biscarboxylates.

4. The process according to claim 2 wherein the palladium based catalyst further contains phosphorus-containing ligands or phosphoms-containing ligands can be added separately to the reaction mixture in step a).

5. The process according to anyone of claims 1-4 wherein the amount of catalyst used in step a) is from 0.001 to 0.5 mol % with respect to the compound (II).

6. The process according to anyone of claims 1-5 wherein R⁴ and R¹ are hydrogen atoms and R³ and R² are linear or branched, cyclic or acyclic, C₁-C₂₀-alkyl radicals.

7. The process according to anyone of claims 1 to 6 wherein step b) comprises the following substeps:
i) contacting the compound of formula (IV) obtained from step a) with two equivalents of a base; and
ii) treating the dianionic compound obtained from step i) with a compound of formula (V) 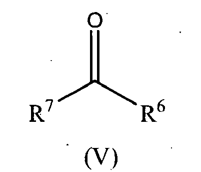 wherein R⁶ and R⁷ equal to or different from each other are selected from the group consisting of hydrogen, chlorine, bromine, iodine, OR and NR₂, wherein R is a C₁-C₂₀ hydrocarbon radical optionally containing heteroatoms belonging to groups 13-17 of the Periodic Table of the Elements.

8. The process according to anyone of claims 1 to 6 wherein step b) comprises the following substeps:
i) contacting the compound of formula (IV) obtained from step a) with two equivalent of a base and subsequently with one equivalent of a compound selected from chlorine, bromine or iodine in order to obtain an anionic monohalogenated derivative; and
ii) treating the an anionic monohalogenated compound obtained from step i) with a compound of formula (Va)
[MₘLⱼ(CO)ₙ]^{a} (Va)
wherein M is a transition metal of groups 4-11 of the periodic table; L is a ligand that coordinates the metal M that can be neutral or with a positive or negative charge; a ranges from -4 to +4 and represents the charge of the complex (when a is 0 the complex is neutral); m ranges from 1 to 20; j ranges from 0 to 30; and n ranges from 1 to 50.

9. The process according to anyone of claims 1 to 8 wherein the base used in step b) is selected from hydroxides and hydrides of alkali- and alkaline-earth metals, metallic sodium and potassium and organometallic lithium compounds.

10. The process according to anyone of claims 1 to 9 wherein the reduction step c) comprises the following substeps:
i) contacting the compound of formula (IVa) described in claim 1 with N₂H₄;
ii) adding a solution of KOH in water; and
iii) filtering the solid and recovering the product.

## Patentansprüche

1. Verfahren zur Herstellung von Cyclopentadienderivaten mit der Formel (I) 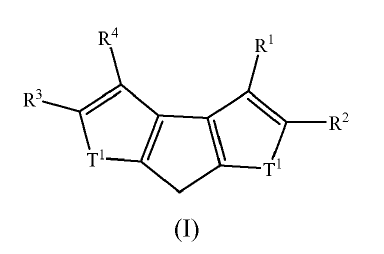 worin
T¹ aus der aus Sauerstoff (O) oder Schwefel (S) bestehenden Gruppe ausgewählt ist; R¹, R², R³ und R⁴, gleich oder voneinander verschieden, Wasserstoffatome oder C₁-C₄₀-Kohlenwasserstoffreste sind, oder R¹ und R² und/oder R³ und R⁴ sich zu ein oder mehr aromatischen oder aliphatischen kondensierten C₃-C₆-Ringen verbinden können;
mit den folgenden Schritten:
a) Umsetzen einer Verbindung der Formel (II) 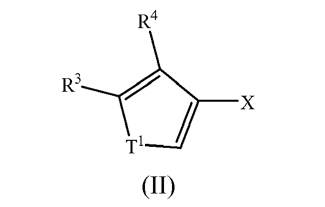 mit einer Verbindung der Formel (III) 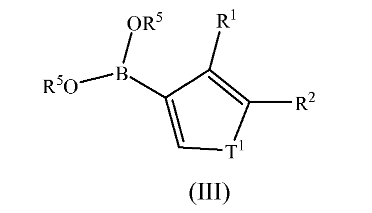 in Gegenwart eines Katalysators auf Palladium- oder Nickelbasis und einer Base;
wobei T¹, R¹, R², R³ und R⁴ die oben angegebene Bedeutung haben, X aus der aus Chlor, Iod und Brom bestehenden Gruppe ausgewählt ist; and R⁵, gleich oder voneinander verschieden, Wasserstoffatome, lineare oder verzweigte, zyklische oder azyklische, C₁-C₂₀-Alkyl-, C₂-C₂₀-Alkenyl-, C₂-C₂₀-Alkynyl-, C₆-C₂₀-Aryl-, C₇-C₂₀-Alkylaryl- oder C₇-C₂₀-Arylalkylreste darstellt;
b) Inkontaktbringen der aus Schritt a) resultierenden Verbindung der Formel (IV) 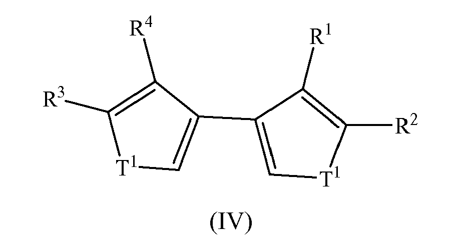 mit einem Carbonylierungssystem, um eine Verbindung der Formel (IVa) zu erhalten 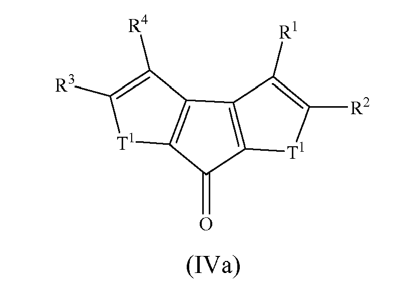 und
c) Behandeln des in Schritt b) erhaltenen Produkts mit einem Reduktionsmittel.

2. Verfahren nach Anspruch 1, wobei in Schritt a) Verbindungen, in denen Palladium im Oxidationszustand (0) vorliegt, als Katalysator verwendet werden.

3. Verfahren nach Anspruch 2, wobei der Katalysator auf Palladiumbasis ausgewählt ist aus Palladiumketonaten, Palladiumacetylacetonaten, Nitrilpalladiumhalogeniden, Palladiumhalogeniden, Allylpalladiumhalogeniden und/oder Palladiumbiscarboxylaten.

4. Verfahren nach Anspruch 2, wobei der Katalysator auf Palladiumbasis ferner phosphorhaltige Liganden enthält oder phosphorhaltige Liganden in Schritt a) dem Reaktionsgemisch getrennt zugesetzt werden können.

5. Verfahren nach einem der Ansprüche 1-4, wobei die in Schritt a) verwendete Menge an Katalysator von 0,001 bis 0,5 mol-% in Bezug auf die Verbindung (II) beträgt.

6. Verfahren nach einem der Ansprüche 1-5, wobei R⁴ und R¹ Wasserstoffatome sind und R³ und R² lineare oder verzweigte, zyklische oder azyklische, C₁-C₂₀-Alkylreste sind.

7. Verfahren nach einem der Ansprüche 1 bis 6, wobei Schritt b) die folgenden Unterschritte umfasst:
i) die aus Schritt a) resultierende Verbindung der Formel (IV) wird mit zwei Äquivalenten einer Base in Kontakt gebracht; und
ii) die aus Schritt i) resultierende dianionische Verbindung wird mit einer Verbindung der Formel (V) behandelt 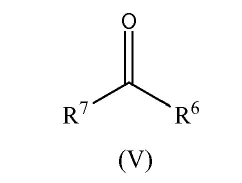 worin R⁶ und R⁷, gleich oder voneinander verschieden, aus der aus Wasserstoff, Chlor, Brom, Iod, OR und NR₂ bestehenden Gruppe ausgewählt sind, wobei R ein C₁-C₂₀-Kohlenwasserstoffrest ist, der optional Heteroatome enthält, die zu den Gruppen 13-17 des Periodensystems der Elemente gehören.

8. Verfahren nach einem der Ansprüche 1 bis 6, wobei Schritt b) die folgenden Unterschritte umfasst:
i) die aus Schritt a) resultierende Verbindung der Formel (IV) wird mit zwei Äquivalenten einer Base und anschließend mit einem Äquivalent einer Verbindung in Kontakt gebracht, die aus Chlor, Brom oder Iod ausgewählt ist, um ein anionisches monohalogeniertes Derivat zu erhalten; und
ii) die aus Schritt i) resultierende anionische monohalogenierte Verbindung wird mit einer Verbindung der Formel (Va) behandelt
[MₘLⱼ(CO)ₙ]^{a} (Va)
worin M ein Übergangsmetall der Gruppen 4-11 des Periodensystems ist; L ist ein Ligand, der sich an das Metall M anlagert, das neutral sein kann oder eine positive oder negative Ladung haben kann; a liegt im Bereich von -4 bis +4 und repräsentiert die Ladung des Komplexes (wenn a 0 ist, dann ist der Komplex neutral); m liegt im Bereich von 1 bis 20; j liegt im Bereich von 0 bis 30; und n liegt im Bereich von 1 bis 50.

9. Verfahren nach einem der Ansprüche 1 bis 8, wobei die in Schritt b) verwendete Base aus Hydroxiden und Hydriden von Alkali- und Erdalkalimetallen, metallischen Natrium- und Kaliumverbindungen sowie metallorganischen Lithiumverbindungen ausgewählt ist.

10. Verfahren nach einem der Ansprüche 1 bis 9, wobei der Reduktionsschritt c) die folgenden Unterschritte umfasst:
i) die in Anspruch 1 beschriebene Verbindung der Formel (IVa) wird mit N₂H₄ in Kontakt gebracht;
ii) es wird eine Lösung von KOH in Wasser zugesetzt; und
iii) der Feststoff wird gefiltert und das Produkt wird gewonnen.

## Revendications

1. Procédé pour préparer des dérivés de cyclopentadiène présentant la formule (I) 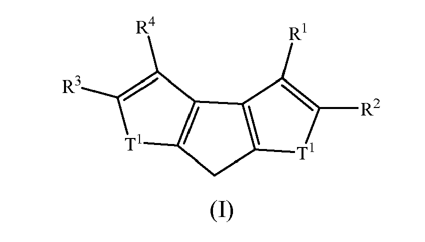 où
T¹ est choisi dans le groupe constitué par oxygène (O) ou soufre (S) ;
R¹, R², R³ et R⁴, identiques ou différents l'un de l'autre, représentent des atomes d'hydrogène, des radicaux hydrocarbonés en C₁-C₄₀ ou R¹ et R² et/ou R³ et R⁴ peuvent s'assembler pour former un ou plusieurs cycles condensés aromatiques ou aliphatiques en C₃-C₆ ;
comprenant les étapes suivantes :
a) réaction d'un composé de formule (II) 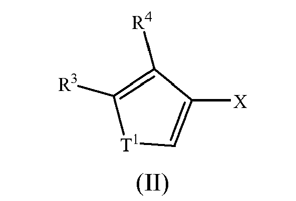 avec un composé de formule (III) 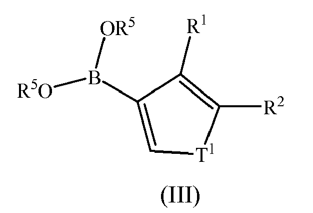 en présence d'un catalyseur à base de palladium ou de nickel et d'une base ;
où T¹, R¹, R², R³ et R⁴ présentent la signification indiquée ci-dessus, X est choisi dans le groupe constitué par chlore, iode, brome ; et R⁵, identique ou différent l'un de l'autre, représente un atome d'hydrogène, des radicaux C₁-C₂₀-alkyle, C₂-C₂₀-alcényle, C₂-C₂₀-alcynyle, linéaires ou ramifiés, cycliques ou acycliques, C₆-C₂₀-aryle, C₇-C₂₀-alkylaryle ou C₇-C₂₀-arylalkyle ;
b) mise en contact du composé de formule (IV) 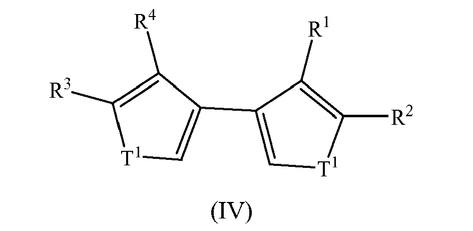 obtenu dans l'étape a) avec un système de carbonylation ; afin d'obtenir un composé de formule (IVa) 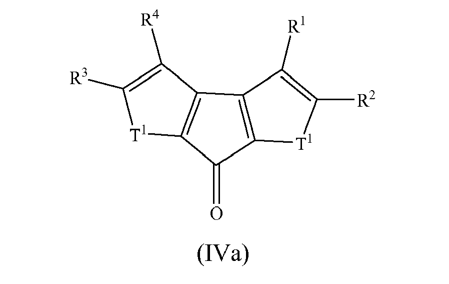 et
c) traitement du produit obtenu dans l'étape b) avec un agent réducteur.

2. Procédé selon la revendication 1, où, dans l'étape a), des composés dans lesquels le palladium est présent dans l'étage d'oxydation (0) sont utilisés comme catalyseur.

3. Procédé selon la revendication 2 où le catalyseur à base de palladium est choisi parmi les cétonates de palladium, les acétylacétonates de palladium, les halogénures de nitrilepalladium, les halogénures de palladium, les halogénures d'allylpalladium et/ou les biscarboxylates de palladium.

4. Procédé selon la revendication 2, où le catalyseur à base de palladium contient en outre des ligands contenant du phosphore ou des ligands contenant du phosphore peuvent être ajoutés séparément au mélange réactionnel dans l'étape a).

5. Procédé selon l'une quelconque des revendications 1-4 où la quantité de catalyseur utilisée dans l'étape a) est de 0,001 à 0,5% en mole par rapport au composé (II).

6. Procédé selon l'une quelconque des revendications 1-5 où R⁴ et R¹ représentent des atomes d'hydrogène et R³ et R² représentent des radicaux C₁-C₂₀-alkyle linéaires ou ramifiés, cycliques ou acycliques.

7. Procédé selon l'une quelconque des revendications 1 à 6, où l'étape b) comprend les sous-étapes suivantes :
i) mise en contact du composé de formule (IV) obtenu dans l'étape a) avec deux équivalents d'une base ; et
ii) traitement du composé dianionique obtenu dans l'étape i) avec un composé de formule (V) : 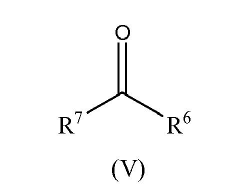 où R⁶ et R⁷, identiques ou différents l'un de l'autre, sont choisis dans le groupe constitué par hydrogène, chlore, brome, iode, OR et NR₂, où R représente un radical hydrocarboné en C₁-C₂₀ contenant éventuellement des hétéroatomes appartenant aux groupes 13-17 du tableau périodique des éléments.

8. Procédé selon l'une quelconque des revendications 1 à 6, où l'étape b) comprend les sous-étapes suivantes :
i) mise en contact du composé de formule (IV) obtenu dans l'étape a) avec deux équivalents d'une base et ensuite avec un équivalent d'un composé choisi parmi le chlore, le brome ou l'iode de manière à obtenir un dérivé monohalogéné anionique ; et
ii) traitement du composé monohalogéné anionique obtenu dans l'étape i) avec un composé de formule (Va) :
[MₘLⱼ(CO)ₙ]^{a} (Va)
où M est un métal de transition des groupes 4-11 du tableau périodique ; L est un ligand qui coordine le métal M qui peut être neutre ou avec une charge positive ou négative ; a vaut -4 à +4 et représente la charge du complexe (lorsque a vaut 0, le complexe est neutre) ; m vaut 1 à 20 ; j vaut 0 à 30 ; et n vaut 1 à 50.

9. Procédé selon l'une quelconque des revendications 1 à 8 où la base utilisée dans l'étape b) est choisie parmi les hydroxydes et les hydrures de métaux alcalins ou alcalino-terreux, le sodium métallique et le potassium métallique et les composés organométalliques du lithium.

10. Procédé selon l'une quelconque des revendications 1 à 9, dans lequel l'étape de réduction c) comprend les sous-étapes suivantes :
i) mise en contact du composé de formule (IVa) décrit dans la revendication 1 avec du N₂H₄;
ii) addition d'une solution de KOH dans l'eau ; et
iii) filtration du solide et récupération du produit.
